# EUROPÄISCHE PATENTSCHRIFT

(11) **EP 1 415 551 B1**
(45) Veröffentlichungstag und Bekanntmachung des Hinweises auf die Patenterteilung: **06.05.2009**
(21) Anmeldenummer: 02024354.9
(22) Anmeldetag: 02.11.2002
(51) Int. Cl.: A23L 3/20, A61L 2/04, A23L 3/22

(54) **Vorrichtung zur Wärmebehandlung eines fliessfähigen Mediums**
Device for the heat treatment of a flowable agent
Dispositif pour le traitement thermique des agents fluides

(43) Veröffentlichungstag der Anmeldung: 06.05.2004
(73) Patentinhaber: Finnatec Prozessanlagen Gmbh, 79865 Grafenhausen (DE)
(72) Erfinder: Finnah, Josef, 49661 Cloppenburg (DE)
(74) Vertreter: Stenger, Watzke & Ring

(56) Entgegenhaltungen:
- EP-A- 0 214 618
- DE-A- 2 929 823
- DE-A- 3 009 889
- US-A- 6 136 362

## Beschreibung

Die Erfindung betrifft eine Vorrichtung zur Wärmebehandlung eines fließfähigen Mediums, insbesondere eines Lebensmittels, mit einer Mehrzahl von strömungstechnischen miteinander verbundenen Wärmeüberträgern, die in Strömungsrichtung des Mediums hintereinander angeordnet sind.

Die Wärmebehandlung von fließfähigen Medien, insbesondere von Lebensmitteln, ist aus dem Stand der Technik bekannt und dient insbesondere dazu, die auf diese Weise behandelten Lebensmittel haltbarer zu machen bzw. eine Haltbarkeitsverlängerung zu erzielen. Bekannte Wärmebehandlungsverfahren sind in diesem Zusammenhang die Thermisierung, die Pasteurisierung, die Ultra-Hocherhitzung oder die Sterilisierung.

Bekannt sind aus der Praxis zwei dem Grunde nach unterschiedliche Wärmebehandlungsverfahren; zum einen das direkte und zum anderen das indirekte Wärmebehandlungsverfahren.

Beim direkten Wärmebehandlungsverfahren wird das zu wärmende Medium direkt z. B. mit Wasser beaufschlagt und so erwärmt. Das vom Medium unter Umständen dabei aufgenommene Wasser als kondensierter Dampf ist dem Medium in einem nachgeschalteten Verfahrensschritt wieder zu entziehen.

Dieses geschieht zumeist in einem Entspannungskühler, in welchem das wärmebehandelte Medium abgekühlt und das dabei entstehende Kondensat bzw. Wasser dem Medium entzogen wird.

Von Nachteil bei dem indirekten Wärmebehandlungsverfahren ist die vorzunehmende Entspannungskühlung, bei der in der Regel eine Schädigung des Mediums auftritt. Dabei erfolgt eine Schädigung nicht im sichtbaren Bereich, sondern im für das menschliche Auge unsichtbaren Bereich, beispielsweise bezüglich der in einem Medium vorhandenen Stabilisatoren oder dergleichen.

Im Unterschied zu dem vorbeschriebenen direkten Wärmebehandlungsverfahren wird bei einem indirekten Wärmebehandlungsverfahren das zu erwärmende Medium nicht direkt dem Wärmemedium, beispielsweise Wasser oder Dampf, ausgesetzt. Eine Übertragung der Wärme findet statt dessen innerhalb eines Wärmeüberträgers, beispielsweise eines Plattenwärmetauschers statt. Von Vorteil bei diesem Verfahren ist, daß keinerlei Dampf, Wasser oder dergleichen Wärmemedien direkt dem zu erwärmenden Medium zuzuführen sind. Mit Nachteil hat sich jedoch herausgestellt, daß sich insbesondere bei einer Erwärmung des Mediums im Bereich der Ultra-Hocherhitzung Ablagerungen im Wärmeüberträger ausbilden, die zur Wahrung der Qualität des wärmebehandelten Mediums in regelmäßigen Abständen zu entfernen sind.

Die Ablagerungen im Wärmeüberträger ergeben sich infolge des Umstands, daß im laufenden Wärmebehandlungsprozeß das in einen Wärmeüberträger eingebrachte Medium stets kühler als das Wärmeüberträgermaterial ist. Insbesondere im Bereich der Ultra-Hocherhitzung ergibt sich dabei ein so hoher Temperaturunterschied zwischen dem Wärmeüberträgermaterial einerseits und dem Medium andererseits, daß es zu sogenannten Anback-Vorgängen innenseitig am Wärmeüberträgermaterial kommen kann. Diese am Wärmeüberträgermaterial verbleibenden Rückstände des wärmebehandelten Mediums sind zur Qualitätssicherung in zum Teil relativ kurz aufeinanderfolgenden zeitlichen Abständen zu entfernen. Eingesetzt werden hierfür spezielle Reinigungsmittel, mit denen die Wärmeüberträger behandelt und gereinigt werden. Während eines solchen Reinigungsprozesses ist die gesamte Anlage in nachteiliger Weise stillzulegen, was je nach Behandlungsdauer zu immensen Produktionsausfällen führen kann. Erst wenn die Reinigung vollständig abgeschlossen und das Reinigungsmittel dem Wärmeüberträger wieder vollständig entzogen wurde, kann ein neuer Wärmebehandlungsprozeß begonnen werden. Im Laufe der weiteren Prozeßdurchführung kommt es dann wieder zu einem Ansetzen von Mediumsbestandteilen an der inneren Oberfläche des Wärmeüberträgers, so daß alsbald wieder eine entsprechende Reinigung durchzuführen ist.

Die Patentschrift DE 2929823 offenbart die Steuerung einer Pasteurisier- und Kühlanlage zur Behandlung eines flüssigen Produkts, indem die Menge des Zubrandes bestimmt wird. Die Anlage ermöglicht einen automatisierten und fembetätigten Betrieb. Eine parallele Anordnung von Wärmeüberträgern ist nicht offenbart.

Ein indirekter Wärmetauscher, der zumindest eine Teilreinigung im laufenden Prozeß ermöglicht, ist aus der DE 30 09 889 A1 bekannt. Es wird hier ein indirekter Wärmetauscher vorgeschlagen, der durch eine Mehrzahl von seriell oder parallel angeordneten Teilwärmetauschem verfügt, von denen mindestens einer jeweils reinigbar ist, während die anderen Teilwärmetauscher in Betrieb sind, wobei diese jeweils insgesamt zumindest für den Gesamtwärmeaustausch ausgelegt sind. So wird mit der DE 30 09 889 A1 beispielsweise ein aus fünf Teilwärmetauschern gebildeter Wärmetauscher vorgeschlagen, wobei vier der Teilwärmetauscher für den angestrebten Gesamtwärmeaustausch ausreichend sind. Einer der Teilwärmetauscher kann sich deshalb in einer Reinigungsstellung befinden, während die verbleibenden vier Teilwärmetauscher in ihre Betriebsstellung verbracht sind. Zur wahlweisen Reinigung eines der fünf Teilwärmetauscher ist vorgesehen, daß ein jeder Teilwärmetauscher über einen verschieblich darin angeordneten Wärmetauscheinsatz verfügt. Dieser ist mittels einer Rahmenkonstruktion verschiebbar in die gewünschte Stellung, das heißt entweder in die Betriebsstellung oder die Reinigungsstellung verfahrbar ausgebildet.

Zur Vermeidung dieses Nachteils ist es **Aufgabe** der Erfindung, eine Vorrichtung zur Wärmebehandlung bereitzustellen, die bei einer gleichzeitig einfachen und sicheren Handhabung einen kontinuierlichen und automatisierten Betriebsablauf ermöglicht.

Zur **Lösung** dieser Aufgabe wird mit der Erfindung eine Vorrichtung vorgeschlagen, bei der zu wenigstens einem Wärmeüberträger ein weiterer, in Strömungsrichtung des Mediums parallel angeordneter Wärmeüberträger vorgesehen ist, wobei beide Wärmeüberträger jeweils an einen ersten Strömungskreislauf für das Medium und an einen zweiten Strömungskreislauf für ein Reinigungsmittel angeschlossen sind und wobei Mittel zum Öffnen und/oder Schließen der Strömungskreisläufe vorgesehen sind, so daß die beiden Wärmeüberträger im gegenseitigen Wechselbetrieb entweder mit dem Medium oder dem Reinigungsmittel betreibbar sind, wobei eine Regelungseinrichtung vorgesehen ist, die mit einer Meßeinrichtung den Strömungsfluß in den Strömungskreisläufen mißt und/oder die Betriebsstellung der Mittel zum Öffnen und/oder Schließen der Strömungskreisläufe erfaßt und ein entsprechendes Signal abgibt, die mit einer Vergleichsschaltung dieses Signal mit wahlweise vorgebbaren Betriebsparametern vergleicht und bei Gleichheit bzw. Ungleichheit eine Umstellung der Mittel zum Öffnen und/oder Schließen der Strömungskreisläufe vornimmt.

Kerngedanke der erfindungsgemäßen Lehre ist die redundante Anordnung wenigstens eines Wärmeüberträgers. Erreicht wird dies durch die Ausbildung zweier in Strömungsrichtung des Mediums parallel angeordneter Wärmeüberträger, die derart strömungstechnisch miteinander verschaltet sind, daß im gegenseitigen Wechsel entweder der eine Wärmeüberträger oder der andere Wärmeüberträger entweder mit zu erwärmendem Medium oder mit Reinigungsmittel durchströmt wird.

In der Konsequenz erlaubt es die erfindungsgemäße Vorrichtung, eine Wärmebehandlungsanlage kontinuierlich zu betreiben. Totzeiten können mithin in vorteilhafter Weise vermieden werden.

Zur Funktion der erfindungsgemäßen Vorrichtung im einzelnen: Die beiden redundant, d.h. parallel angeordneten Wärmeüberträger sind einerseits an einen Strömungskreislauf für das zu erwärmende Medium und andererseits an einen Strömungskreislauf für das Reinigungsmittel angeschlossen. Beide Strömungskreisläufe können miteinander über beispielsweise Mehrwegventilen gekoppelt sein, wobei die Verkopplung derart ausgebildet ist, daß ein Wärmeüberträger entweder nur mit dem wärmezubehandelnden Medium oder nur mit dem Reinigungsmittel betrieben werden kann. Vorzugsweise sind in den Leitungen der einzelnen Strömungskreisläufe Sperr- bzw. Sicherheitsventile vorhanden, so daß es auch bei einem Defekt eines im System befindlichen Ventils nicht in unerwünschter Weise dazu kommen kann, daß eine Vermischung von zu erwärmendem Medium und Reinigungsmittel eintritt. Eine solche Sicherheitsanordnung ist dabei auch insofern von Vorteil, als daß Beschädigungen beispielsweise im Leitungssystem oder bezüglich verwendeter Dichtungen vorgebeugt ist.

Im laufenden Betrieb der Vorrichtung wird einer der beiden Wärmeüberträger an den Strömungskreislauf für das zu erwärmende Medium und der andere Wärmeüberträger an den Kreislauf für das Reinigungsmittel angeschlossen. Während das zu erwärmende Medium den einen Wärmeüberträger durchströmt und dabei erhitzt wird, erfolgt zeitgleich in dem parallel geschalteten Wärmeüberträger eine Reinigung desselben. Reinigungsvorgang in dem einen Wärmeüberträger und Erwärmung des zu erwärmenden Mediums in dem anderen Wärmeüberträgern sind dabei zeitlich derart aufeinander abgestimmt, daß eine Reinigung des ersten Wärmeüberträgers abgeschlossen ist, sobald der zweite Wärmeüberträger soweit mit abgesetztem, d.h. an den Innenoberflächen angebackenem Material verunreinigt ist, daß eine Weiterbetreibung dieses Wärmeüberträgers nur noch mit einem Qualitätsverlust des zu erwärmenden Mediums möglich wäre. In diesem Moment erfolgt sodann eine Umstellung zwischen den beiden Wärmeüberträgern. Der verunreinigte Wärmeüberträger wird durch Ansteuern der Mehrwegventile aus dem Strömungskreislauf für das zu erwärmende Medium herausgenommen, während der gereinigte und bereitstehende Wärmeüberträger in diesen Strömungskreislauf hineingeschaltet wird. Der fortlaufende Wärmebehandlungsprozeß findet nunmehr über den neu in das System eingeschalteten Wärmeüberträger statt. Der verunreinigte Wärmeüberträger wird indes in den Strömungskreislauf für das Reinigungsmittel eingeschaltet. Dieser wird alsdann vom Reinigungsmittel durchströmt und die Innenoberfläche des Wärmeüberträgers wird entsprechend gereinigt. Sobald der Reinigungsvorgang abgeschlossen ist steht auch dieser Wärmeüberträger wieder für eine Wärmebehandlung des Mediums bereit und wird in den kontinuierlichen Wärmebehandlungsprozeß eingeschaltet, sobald der andere Wärmeüberträger soweit verschmutzt ist, daß auch dieser einer erneuten Reinigung zu unterziehen ist.

In vorteilhafter Weise erlaubt die erfindungsgemäße Vorrichtung die kontinuierliche Betreibung eines Wärmebehandlungsprozesses. Totzeiten infolge eines erforderlichen Reinigungsvorganges können vermieden werden, da die redundante Anordnung der beiden parallel geschalteten Wärmeüberträger die Wärmebehandlung des zu erwärmenden Mediums mittels des einen Wärmeüberträgers bei gleichzeitiger Reinigung des anderen Wärmeüberträgers ermöglicht. Für den Wärmebehandlungsprozeß steht mithin immer ein gereinigter und einsatzfähiger Wärmeüberträger zur Verfügung.

Gemäß einem Merkmal der Erfindung sind die Wärmeüberträger im Kreuzstrom und/oder im Gegenstrom betreibbar. Je nach zu erwärmendem Medium eignet sich hierbei entweder die eine oder die andere Betreibungsart, wobei sich in der Praxis insbesondere das Betreiben im Gegenstrom bewährt hat.

Gemäß einem weiteren Merkmal der Erfindung sind innerhalb der Strömungskreisläufe Sperrventile angeordnet. Diese Ausgestaltungsform der Erfindung dient insbesondere der Sicherheit des Systems gegenüber Leckagen. Auf diese Weise kann nämlich gewährleistet werden, daß es auch bei Leckagen infolge von beispielsweise Dichtungsundichtigkeiten nicht zu einer ungewollten Vermischung von Reinigungsmittel einerseits und zu erwärmendem Medium andererseits kommt.

Erfindungsgemäß ist eine Regelungseinrichtung vorgesehen, die mit einer Meßeinrichtung den Strömungsfluß in den Strömungskreisläufen mißt und/oder die Betriebsstellung der Mittel zum Öffnen und/oder Schließen der Strömungskreisläufe erfaßt und ein entsprechendes Signal abgibt, die mit einer Vergleichsschaltung dieses Signal mit wahlweise vorgebbaren Betriebsparametern vergleicht und bei Gleichheit bzw. Ungleichheit eine Umstellung der Mittel zum Öffnen und/oder Schließen der Strömungskreisläufe vornimmt.

Die Anordnung einer solchen Regelungseinrichtung ermöglicht in vorteilhafter Weise einen automatisierten Betrieb. Die gesamte Wärmebehandlungsanlage kann auf diese Weise ferngesteuert und die redundant angeordneten Wärmeüberträger können so in Abhängigkeit der wahlweise vorgebbaren Betriebsparameter in den Prozeß eingeschaltet bzw. zur Reinigungsprozessen aus dem Prozeß herausgeschaltet werden. Wahlweise vorgebbare Betriebsparameter sind beispielsweise die Temperatur des Mediums, die Temperatur des Wärmeüberträgers, die verstrichene Zeit seit letztmalig erfolgter Reinigung des Wärmeüberträgers, die Schichtdicke oder Vollständigkeit der sich an der Innenoberfläche des Wärmeüberträgers absetzenden Verunreinigung oder die seit dem letzten Wechsel zwischen den beiden redundant angeordneten Wärmeüberträgern verstrichene Zeit.

Vorgeschlagen wird mit der Erfindung des weiteren ein Verfahren zur Wärmebehandlung eines fließfähigen Mediums, insbesondere eines Lebensmittels, bei dem das Medium im gegenseitigen Wechselbetrieb mit einem Reinigungsmittel in Strömungsrichtung des Mediums parallel angeordnete Wärmeüberträger durchströmt. Vorzugsweise wird dieses Verfahren mit der erfindungsgemäßen Vorrichtung durchgeführt.

Zum Zwecke des Wärmeaustausches kann gemäß einem weiteren Merkmal der Erfindung vorgesehen sein, daß das bereits wärmebehandelte Medium im Gegenstrom am noch nicht wärmebehandelten Medium vorbeigeführt wird. Diese Verfahrensdurchführung dient insbesondere der Wärme-, d.h. Energierückgewinnung.

Die im Verfahren verwendeten Wärmeüberträger werden vorzugsweise als Platten- oder Röhrenwärmetauscher ausgebildet und mit einer Temperatur von 6°C bis 300°C, vorzugsweise von 6°C bis 250°C, betrieben.

Weitere Merkmale und Vorteile der Erfindung ergeben sich aus der Beschreibung anhand der Figuren. Dabei zeigen:
- Fig. 1:: in einer schematischen Übersichtsdarstellung den Aufbau der erfindungsgemäßen Vorrichtung und
- Fig. 2:: in einer schematischen Übersichtsdarstellung die redundante Anordnung der Wärmeüberträger im Detail.

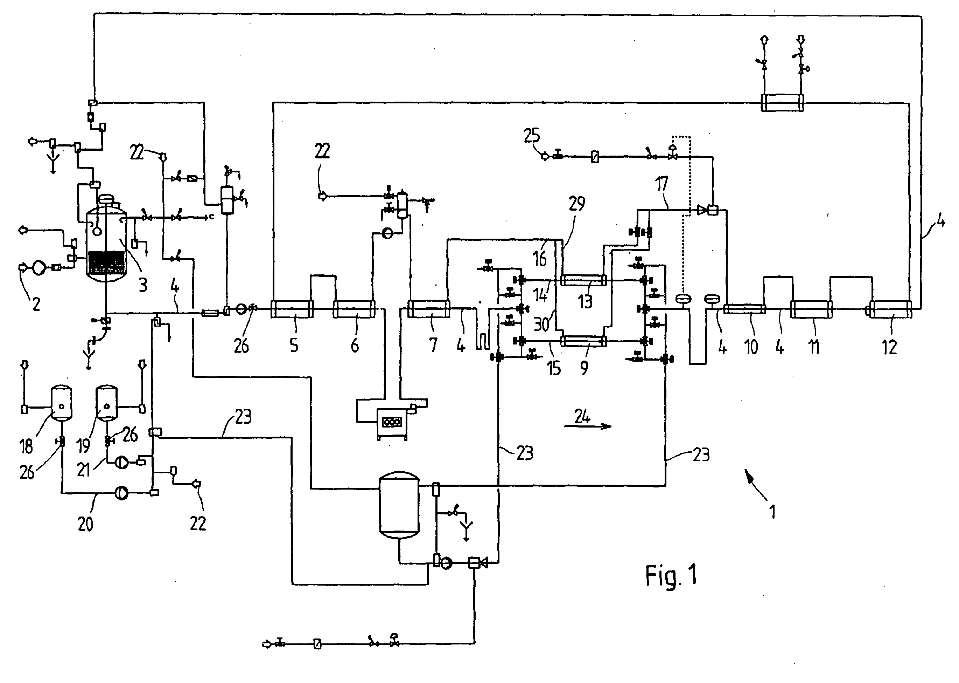

Fig. 1 zeigt gemäß einer schematischen Übersichtsdarstellung die erfindungsgemäße Vorrichtung 1 zur Wärmebehandlung eines fließfähigen Mediums, insbesondere eines fließfähigen Lebensmittels. Das fließfähige Medium, nachfolgend Produkt genannt, gelangt über den Produkteinlaß 2 in die Wärmebehandlungsvorrichtung 1. Vorgesehen ist ein Reservoir 3 über das das Produkt in die den ersten Strömungskreislauf bildende Leitung 4 hineingeführt wird. Über die Leitung 4 wird das wärmezubehandelnde Medium unter Zwischenschaltung von Sperrventilen 26 einer Mehrzahl von in Strömungsrichtung 24 des Mediums hintereinander geschalteten Wärmeüberträgern 5, 6, 7, 9, 10, 11, 12 zugeführt. Den in Strömungsrichtung 24 letzten Wärmeüberträger 12 verläßt über die Leitung 4 sodann ein vollständig wärmebehandeltes Produkt.

Der Wärmeüberträger 9 dient der Ultra-Hocherhitzung des Produktes, wobei Temperaturen in einem Bereich von 60°C bis 250°C für das Produkt zu erreichen sind. Das sich im kontinuierlichen Fluß befindliche Produkt strömt in den Wärmeüberträger 9 hinein und weist zu Beginn der Behandlung eine gegenüber dem Wärmeüberträgermaterial deutlich geringere Temperatur auf, was dazu führt, daß es bei einer Berührung des Produktes mit der Innenoberfläche des Wärmeüberträgermaterials des Wärmeüberträgers 9 zu einem "Anbacken" des Produkts an der Innenoberfläche des Wärmeüberträgers 9 kommt. Diese durch das Anbacken des Produktes bedingten Verunreinigungen sind in regelmäßigen Abständen durch Reinigen zu entfernen, da es ansonsten zu Qualitätseinbußen hinsichtlich des wärmebehandelten Produktes kommt.

Für die Reinigung des Wärmeüberträgers 9 wird diesem über die Zuführungsleitung 23 ein Reinigungsmittel zugeführt. Dieses Reinigungsmittel kann bedarfsgerecht zusammengemischt werden. Zur Verfügung stehen eine im Laugenreservoir 18 befindliche Lauge sowie eine im Säurereservoir 19 befindliche Säure. Diese werden über die Leitung 20 und 21 mit über den Wasserzufluß 22 einströmenden Wassers bedarfsgerecht verdünnt. Zur Absicherung des Leitungssystems sind Sperrventile 26 vorgesehen.

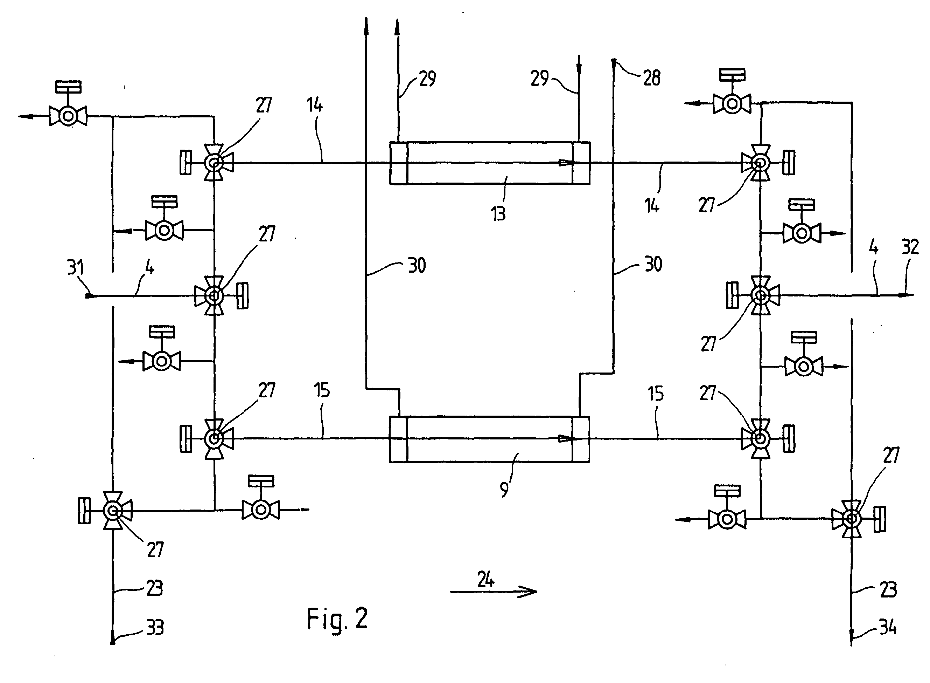

Für eine Reinigung des Wärmeüberträgers 9 wird diesem das Reinigungsmittel über die Leitung 23 zugeführt, wobei während des Reinigungsprozesses eine Wärmebehandlung des Produktes durch diesen Wärmeüberträger nicht möglich ist. Erfindungsgemäß erfolgt während eines Reinigungsvorganges für den Wärmeüberträger 9 eine Wärmebehandlung des Produktes mittels des Wärmeüberträgers 13. Dieser ist in Strömungsrichtung 24 zum Wärmeüberträger 9 parallel angeordnet und über die Leitungen 14 und 15 sowie der zwischengeschalteten Mehrwegventile 27 derart redundant zum Wärmeüberträger 9 geschaltet, daß die beiden Wärmeüberträger 9 und 13 im gegenseitigen Wechselbetrieb entweder mit Reinigungsmittel oder mit dem zu erwärmenden Produkt betrieben werden können. Eine nähere Darstellung dieser parallelen Anordnung von Wärmeüberträger 9 und Wärmeüberträger 13 zeigt Fig. 2. Gezeigt sind hier der Wärmeüberträger 9 sowie der Wärmeüberträger 13, die in Strömungsrichtung 24 parallel zueinander angeordnet sind. Über die Leitung 30 wird dem Wärmeüberträger 9 ein Wärmemedium 28, beispielsweise Dampf, der über den Dampfeinlaß 25 in das System gelangt, zugeführt. Der Wärmeüberträger 13 wird gleichfalls mit einem Wärmemedium, beispielsweise Dampf beheizt, wobei hier für eine Zuführung die Leitung 29 vorgesehen ist.

Das wärmezubehandelnde Produkt wird über den Produkteintritt 31 und die Leitung 4 entweder dem Wärmeüberträger 9 oder dem Wärmeüberträger 13 zugeführt, je nachdem wie die zwischengeschalteten Mehrwegventile 27 ausgerichtet sind. Erfolgt ein Strömungsfluß des Produktes über den Wärmeüberträger 13, d.h. durch die Leitung 14, so ist die Mehrwegventilschaltung dergestalt, daß die Leitung 15 nicht vom Produkt durchströmt wird, d.h. der Wärmeüberträger 9 nicht mit dem Produkt beaufschlagt wird. Das die Wärmeüberträger 9 bzw. 13 verlassende, wärmebehandelte Produkt tritt über die Leitung 4 am Produktaustritt 32 aus.

Zur Reinigung der beiden Wärmeüberträger 9 und 13 wird über die Zuführungsleitung 23 ein Reinigungsmittel zugeführt, das über den Reinigungsmitteleintritt 33 in das System gelangt. Je nachdem wie die Mehrwegventile 27 geschaltet sind, durchströmt das Reinigungsmittel entweder die Leitung 14 oder die Leitung 15 und säubert mithin entweder den Wärmeüberträger 13 oder den Wärmeüberträger 9. Wenn das Produkt über die Zuführungsleitung 14 dem Wärmeüberträger 13 zugeführt wird, ist diese Leitung für das Reinigungsmittel gesperrt, so daß dieses ausschließlich nur durch die Leitung 15 und damit den Wärmeüberträger 9 durchströmen kann. Nach einem Verlassen des Wärmeüberträgers 9 strömt das Reinigungsmittel sodann über die Leitung 23 zum Reinigungsmittelaustritt 34.

Die parallel und redundant verschaltete Anordnung der beiden Wärmeüberträger 9 und 13 ermöglicht in vorteilhafter Weise eine kontinuierliche Wärmebehandlung, wobei diese entweder in dem Wärmeüberträger 9 oder dem Wärmeüberträger 13 durchgeführt wird, während gleichzeitig der sich nicht im Betrieb befindliche Wärmeüberträger gereinigt wird.

Die Wärmezufuhr für die einzelne Wärmeüberträger 5 bis 13 kann entweder mittels Wasser oder Dampf erfolgen, wobei die Wärmeüberträger 9 und 13 vorzugsweise über die Leitung 17 mittels Dampf beaufschlagt werden, der nach einem Durchlaufen durch die beiden Wärmeüberträger 9 und 13 über die gemeinsame Leitung 16 einer Wärmerückgewinnung über Wärmeüberträger 7, 6, 5, 12, 11 und 10 zugeführt wird.

### Bezugszeichenliste

- 1: Wärmebehandlungsvorrichtung
- 2: Produkteinlaß
- 3: Reservoir
- 4: Leitung
- 5 bis 7: Wärmeüberträger
- 9 bis 13: Wärmeüberträger
- 14 bis 17: Leitung
- 18: Laugenreservoir
- 19: Säurereservoir
- 20 bis 21: Leitung
- 22: Wasserzufluß
- 23: Leitung
- 24: Strömungsrichtung
- 25: Dampfeinlaß
- 26: Sperrventil
- 27: Mehrwegventil
- 28: Wärmemedium
- 29 bis 30: Leitung
- 31: Produkteintritt
- 32: Produktaustritt
- 33: Reinigungsmitteleintritt
- 34: Reinigungsmittelaustritt

## Patentansprüche

1. Vorrichtung zur Wärmebehandlung eines fließfähigen Mediums, insbesondere eines Lebensmittels, mit einer Mehrzahl von strömungstechnisch miteinander verbundenen Wärmeüberträgern (5 bis 7 und 9 bis 12), die in Strömungsrichtung (24) des Mediums hintereinander angeordnet sind, wobei zu wenigstens einem Wärmeüberträger (9) ein weiterer, in Strömungsrichtung (24) des Mediums parallel angeordneter Wärmeüberträger (13) vorgesehen ist, wobei beide Wärmeüberträger (9, 13) jeweils an einen ersten Strömungskreislauf (4) für das Medium und an einen zweiten Strömungskreislauf (23) für ein Reinigungsmittel angeschlossen sind und wobei Mittel (27) zum Öffnen und/oder Schließen der Strömüngskreisläufe (4, 23) vorgesehen sind, so daß die beiden Wärmeüberträger (9, 13) im gegenseitigen Wechselbetrieb entweder mit dem Medium oder dem Reinigungsmittel betreibbar sind, wobei eine Regelungseinrichtung vorgesehen ist, die mit einer Meßeinrichtung den Strömungsfluß in den Strömungskreisläufen (4, 23) mißt und/oder die Betriebsstellung der Mittel (27) zum Öffnen und/oder Schließen der Strömungskreisläufe (4, 23) erfaßt und ein entsprechendes Signal abgibt, die mit einer Vergleichsschaltung dieses Signal mit wahlweise vorgebbaren Betriebsparametern vergleicht und bei Gleichheit bzw. Ungleichheit eine Umstellung der Mittel (27) zum Öffnen und/oder Schließen der Strömungskreisläufe vornimmt.

2. Vorrichtung nach Anspruch 1, **dadurch gekennzeichnet, daß** die Wärmeüberträger (5 bis 7 und 9 bis 13) im Kreuzstrom und/oder im Gegenstrom betreibbar sind.

3. Vorrichtung nach Anspruch 1 oder Anspruch 2, **dadurch gekennzeichnet, daß** die Wärmeüberträger (5 bis 7 und 9 bis 13) Platten- oder Röhrenwärmetauscher sind.

4. Vorrichtung nach einem der Ansprüche 1, 2 oder 3, **dadurch gekennzeichnet, daß** die Mittel (27) zum Öffnen und/oder Schließen der Strömungskreisläufe (4, 23) Mehrwegventile sind.

5. Vorrichtung nach einem der vorhergehenden Ansprüche, **dadurch gekennzeichnet, daß** der erste Strömungskreislauf (4) und der zweite Strömungskreislauf (23) unter Zwischenordnung der Mehrwegventile (27) strömungstechnisch miteinander verbunden sind.

6. Vorrichtung nach einem der vorhergehenden Ansprüche, **dadurch gekennzeichnet, daß** innerhalb der Strömungskreisläufe (4, 23) Sperrventile (26) angeordnet sind.

7. Verfahren zur Wärmebehandlung eines fließfähigen Mediums, insbesondere eines Lebensmittels, bei dem das Medium im gegenseitigen Wechselbetrieb mit einem Reinigungsmittel (2) in Strömungsrichtung (24) des Mediums parallel angeordnete Wärmeüberträger (9, 13) durchströmt, wobei eine Regelungseinrichtung vorgesehen ist, die mit einer Meßeinrichtung den Strömungsfluß in den Strömungskreisläufen (4, 23) mißt und/oder die Betriebsstellung der Mittel (27) zum Öffnen und/oder Schließen der Strömungskreisläufe (4, 23) erfaßt und ein entsprechendes Signal abgibt, die mit einer Vergleichsschaltung dieses Signal mit wahlweise vorgebbaren Betriebsparametern vergleicht und bei Gleichheit bzw. Ungleichheit eine Umstellung der Mittel (27) zum Öffnen und/oder Schließen der Strömungskreisläufe vornimmt.

8. Verfahren nach Anspruch 7, **dadurch gekennzeichnet, daß** die Wärmeüberträger (9, 13) im Kreuzstrom und/oder im Gegenstrom betrieben werden.

9. Verfahren nach Anspruch 7 oder 8, **dadurch gekennzeichnet, daß** die Wärmeüberträger (9, 13) mit einer Temperatur von 60°C bis 300°C, vorzugsweise von 60°C bis 250°C, betrieben werden.

10. Verfahren nach Anspruch 9, **dadurch gekennzeichnet, daß** die Wärmeüberträger (9, 13) mit einer Temperatur von 90°C bis 140°C betrieben werden.

11. Verfahren nach einem der Ansprüche 7 bis 9, **dadurch gekennzeichnet, daß** das bereits wärmebehandelte Medium im Gegenstrom am noch nicht wärmebehandelten Medium zum Zwecke des Wärmeaustausches vorbeigeführt wird.

## Claims

1. A device for heat-treating a free-flowing medium, particularly a food, with a plurality of heat exchangers (5 to 7 and 9 to 12) that are fluidically connected to one another and arranged in succession referred to the flow direction (24) of the medium, wherein another heat exchanger (13) is arranged parallel to at least one heat exchanger (9) referred to the flow direction (24) of the medium, wherein both heat exchangers (9, 13) are respectively connected to a first flow circuit (4) for the medium and to a second flow circuit (23) for a cleaning agent, and wherein means (27) are provided for opening and/or closing the flow circuits (4, 23) such that the two heat exchangers (9, 13) can be operated either with the medium or the cleaning agent in a mutually alternating fashion, wherein a control device is provided that measures the flow in the flow circuits (4, 23) and/or determines the operating position of the means (27) for opening and/or closing the flow circuits (4, 23) and generates a corresponding signal, and wherein it compares this signal with selectively presettable operating parameters by means of a comparison circuit and respectively changes over the means (27) for opening and/or closing the flow circuits if the comparison results in a match or mismatch.

2. The device according to Claim 1, **characterized in that** the heat exchangers (5 to 7 and 9 to 13) can be operated in a cross-current and/or in a counter-current.

3. The device according to Claim 1 or Claim 2, **characterized in that** the heat exchangers (5 to 7 and 9 to 13) consist of plate heat exchangers or tubular heat exchangers.

4. The device according to one of Claims 1, 2 or 3, **characterized in that** the means (27) for opening and/or closing the flow circuits (4, 23) consist of multiple-way valves.

5. The device according to one of the preceding claims, **characterized in that** the first flow circuit (4) and the second flow circuit (23) are fluidically connected to one another by means of the multiple-way valves (27).

6. The device according to one of the preceding claims, **characterized in that** shut-off valves (26) are arranged within the flow circuits (4, 23).

7. A method for heat-treating a free-flowing medium, particularly a food, in which the medium and a cleaning agent (2) flow through heat exchangers (9, 13) that are arranged parallel referred to the flow direction (24) of the medium in a mutually alternating fashion, wherein a control device is provided that measures the flow in the flow circuits (4, 23) and/or determines the operating position of the means (27) for opening and/or closing the flow circuits (4, 23) and generates a corresponding signal, and wherein it compares this signal with selectively presettable operating parameters by means of a comparison circuit and respectively changes over the means (27) for opening and/or closing the flow circuits if the comparison results in a match or mismatch.

8. The method according to Claim 7, **characterized in that** the heat exchangers (9, 13) can be operated in a cross-current and/or in a counter-current.

9. The method according to Claim 7 or 8, **characterized in that** the heat exchangers (9, 13) are operated with a temperature of 60°C to 300°C, preferably 60°C to 250°C.

10. The method according to Claim 9, **characterized in that** the heat exchangers (9, 13) are operated with a temperature of 90°C to 140°C.

11. The method according to one of Claims 7 to 9, **characterized in that** the already heat-treated medium is conveyed past the not yet heat-treated medium in a counter-current for heat-exchanging purposes.

## Revendications

1. Dispositif pour le traitement thermique d'un milieu fluide, en particulier d'une denrée alimentaire, comprenant une pluralité de caloporteurs (5 à 7 et 9 à 12) reliés les uns aux autres par écoulement de fluide, qui sont disposés les uns derrière les autres dans la direction d'écoulement (24) du milieu, un autre caloporteur (13) disposé en parallèle dans la direction d'écoulement (24) du milieu étant prévu pour au moins un caloporteur (9), les deux caloporteurs (9, 13) étant raccordés respectivement à un premier circuit d'écoulement (4) pour le fluide et à un second circuit d'écoulement (23) pour un produit de nettoyage et des moyens (27) étant prévus pour l'ouverture et/ou la fermeture des circuits d'écoulement (4, 23), de sorte que les deux caloporteurs (9, 13) peuvent être exploités dans le mode alternatif réciproque soit avec le milieu soit avec le produit de nettoyage, un dispositif de réglage étant prévu, lequel mesure avec un appareil de mesure le flux d'écoulement dans les circuits d'écoulement (4, 23) et/ou enregistre la position de service des moyens (27) pour l'ouverture et/ou la fermeture des circuits d'écoulement (4, 23) et émet un signal approprié, lequel dispositif compare avec un circuit de comparaison ce signal avec des paramètres de service prédéfinissables au choix et effectue en cas d'égalité ou de non égalité une inversion de marche des moyens (27) pour l'ouverture et/ou la fermeture des circuits d'écoulement.

2. Dispositif selon la revendication 1, **caractérisé en ce que** les caloporteurs (5 à 7 et 9 à 13) peuvent être exploités en flux croisé ou en flux opposé.

3. Dispositif selon la revendication 1 ou la revendication 2, **caractérisé en ce que** les caloporteurs (5 à 7 et 9 à 13) sont des échangeurs de chaleur à plaques ou des échangeurs tubulaires.

4. Dispositif selon l'une quelconque des revendications 1, 2 ou 3, **caractérisé en ce que** les moyens (27) pour l'ouverture et/ou la fermeture des circuits d'écoulement (4, 23) sont des distributeurs multivoies.

5. Dispositif selon l'une quelconque des revendications précédentes, **caractérisé en ce que** le premier circuit d'écoulement (4) et le second circuit d'écoulement (23) sont reliés l'un à l'autre au niveau de l'écoulement par l'intercalation des distributeurs multivoies (27).

6. Dispositif selon l'une quelconque des revendications précédentes, **caractérisé en ce que** des vannes d'arrêt (26) sont disposées à l'intérieur des circuits d'écoulement (4, 23).

7. Procédé pour le traitement thermique d'un milieu fluide, en particulier d'une denrée alimentaire, dans lequel le milieu traverse des caloporteurs (9, 13), disposés parallèlement dans le mode alternatif réciproque avec un produit de nettoyage (2) dans le sens d'écoulement (24) du milieu, un dispositif de réglage étant prévu, lequel mesure avec un appareil de mesure le flux d'écoulement dans les circuits d'écoulement (4, 23) et/ou enregistre la position de service des moyens (27) pour l'ouverture et/ou la fermeture des circuits d'écoulement (4, 23) et émet un signal correspondant, lequel dispositif compare avec un circuit de comparaison ce signal avec des paramètres de service prédéfinissables au choix et effectue en cas d'égalité ou de non égalité une inversion de marche des moyens (27) pour l'ouverture et/ou la fermeture des circuits d'écoulement.

8. Procédé selon la revendication 7, **caractérisé en ce que** les caloporteurs (9, 13) sont exploités en flux croisé et/ou en flux opposé.

9. Procédé selon la revendication 7 ou 8, **caractérisé en ce que** les caloporteurs (9, 13) sont exploités avec une température de 60°C à 300°C, de préférence de 60°C à 250°C.

10. Procédé selon la revendication 9, **caractérisé en ce que** les caloporteurs (9, 13) sont exploités avec une température de 90°C à 140°C.

11. Procédé selon l'une quelconque des revendications 7 à 9, **caractérisé en ce que** le milieu déjà traité thermiquement est guidé dans le flux opposé en passant devant le milieu non encore traité thermiquement pour l'échange de chaleur.
